# EUROPÄISCHE PATENTSCHRIFT

(11) **EP 2 046 732 B1**
(45) Veröffentlichungstag und Bekanntmachung des Hinweises auf die Patenterteilung: **09.09.2015**
(21) Anmeldenummer: 07785614.4
(22) Anmeldetag: 10.07.2007
(51) Int. Cl.: C07C 259/12, A61K 31/155

(54) **VERBESSERUNG DER BIOVERFÜGBARKEIT VON WIRKSTOFFEN MIT AMIDINFUNKTION IN ARZNEIMITTELN**
IMPROVEMENT OF THE BIOAVAILABILITY OF ACTIVE SUBSTANCES HAVING AN AMIDINE FUNCTION IN MEDICAMENTS
AMELIORATION DE LA BIODISPONIBILITE DE PRINCIPES ACTIFS À FONCTION AMIDINE DANS DES MEDICAMENTS

(30) Priorität: 21.07.2006 DE 102006034256
(43) Veröffentlichungstag der Anmeldung: 15.04.2009
(73) Patentinhaber: Dritte Patentportfolio Beteiligungsgesellschaft mbH & Co. KG, 12529 Schönefeld/OT Waltersdorf (DE)
(72) Erfinder: CLEMENT, Bernd, 24106 Kiel (DE); REEH, Christiane, 20251 Hamburg (DE)
(74) Vertreter: Zech, Stefan Markus
(86) Internationale Anmeldenummer: PCT/DE2007/001216
(87) Internationale Veröffentlichungsnummer: WO 2008/009264

(56) Entgegenhaltungen:
- RIGGS ET AL: "Factor VIIa inhibitors: A prodrug strategy to improve oral bioavailability" BIOORGANIC & MEDICINAL CHEMISTRY LETTERS, OXFORD, GB, Bd. 16, Nr. 8, 15. April 2006 (2006-04-15), Seiten 2224-2228, XP005323552 ISSN: 0960-894X

## Beschreibung

Die vorliegende Erfindung betrifft die Verbesserung der Bioverfügbarkeit von Arzneistoffen, die wenigstens eine Amidinfunktion aufweisen und entsprechend modifizierte Arzneistoffe enthaltende Arzneimittel.

Pharmazeutische Zubereitungen, die einen Wirkstoff mit einer oder mehreren Amidinfunktionen enthalten, zeigen bei oraler Anwendung nahezu keine pharmakologische Wirkung. Die Voraussetzung für einen therapeutischen Effekt eines Wirkstoffes nach oraler Gabe stellt dessen Aufnahme aus dem Gastrointestinaltrakt dar. Der wichtigste Mechanismus eines solchen Effektes ist die passive Diffusion. Der Grad der Resorption auf dem Weg der passiven Diffusion ist abhängig von der Lipophilie und somit auch abhängig von der Azidität und der Basizität des Wirkstoffes.

Eine stark basische Verbindung wie das Benzamidin liegt im Magen (pH 1) und im Dünndarm (pH 6,4) nahezu vollständig ionisiert vor. Eine Resorption nach oraler Gabe, die den Durchtritt durch die Lipiddoppelschichten der Membranen des Gastrointestinaltraktes erfordert, erfolgt daher nur in sehr geringem Ausmaß. Es ist zu vermuten, dass alle Wirkstoffe die als funktionelle Gruppe ein Amidin aufweisen, eine ungenügende Resorption bei der oralen Anwendung zeigen.

Die Publikation RIGGS ET AL: "Factor VIIa inhibitors: A prodrug strategy to impruve oral bioavailabitity" BIOORGANIC & MEDICAL CHEMISTRY LETTERS, OXFORD, GB, Bd. 16, Nr. 8, 15. April 2006 (2006-04-15), Seiten 2224-2228 beschreibt Amidoxime als mögliche Prodrugs.

Die *N*-hydroxylierten Derivate, wie die Amidoxime, zeigen durch die Einführung des Sauerstoffatoms eine geringere Basizität. Unter physiologischen Bedingungen liegen die Amidoxime nicht protoniert vor. Benzamidoxim stellt eine Modellverbindung für viele Arzneistoffe dar, die eine Amidoximfunktion enthalten [Clement, B. (2002) Drug Met. Rev. 34 565-579]. Bei dem Ximelagatran konnte die orale Bioverfügbarkeit durch Einführung der Amidoximfunktion im Vergleich zum Melagatran jedoch nur von 7% auf 14% gesteigert werden [Clement, B.; Lopian, K. (2003) Drug Met. Dispos. 31 645-651]. Es besteht daher noch immer ein dringender Bedarf an Arzneistoffen mit Amidinfunktion die nach oraler Verabreichung effektiv über den Gastrointestinaltrakt resorbiert werden.

Daher ist es die Aufgabe der vorliegenden Erfindung, die orale Bioverfügbarkeit von Substanzen, die eine Amidinfunktion enthalten, zu erhöhen.

Die Aufgabe wird erfindungsgemäß durch die Merkmale des Anspruchs 1 gelöst. Die Unteransprüche geben vorteilhafte Ausgestaltungen der Erfindung wieder.

Gemäß der erfindungsgemäßen Verwendung wird durch Substitution wenigstens einer Amidiufunktion durch N,N'-Dihydroxyamidine erreicht, dass diese nach oraler Verabreichung zunächst effektiv resorbiert und anschließend durch körpereigene Esterasen und N-Reduktion wieder in die eigentlichen Wirkformen, die Amidine zurückverwandelt werden (Prodrug-Prinzip). Die ausgezeichnete Resorbierbarkeit der abgewandelten Amidinfunktion im Gastrointestinaltrakt ist offensichtlich auf die stark verminderte Basizität und die erhöhte Lipohille der Wirkstolfmoleküle zurückzuführen. Mit der chemischen Abwandlung der Amidinfunktionen zur N,N'-Dihydroxyamidinfunktion wird per pKs-Wert des Amidins von ca. 11 auf den des N,N'-Dihydroxyamidins von ca. 4 verringert. Im Darm, dem Hauptresorptionsort für Wirkstoffe, liegt somit das N,N'-Dihydroxyamidin nahezu vollständig in Form der freien Base vor. Parallel zur Abnahme der Basizität nimmt 'durch die vorgenommene Abwandlung der Amidinfunktion die Lipophilie der 'entsprechenden Wirkstoffe zu.

Es ist ausreichend, wenn der Wirkstoff mindestens eine wirksame Amidingruppe in der vorgeschlagenen Form enthält. Der Wirkstoff kann demnach mehrere Amidingruppen (z.B. zwei wie bei dem Pentamidin) enthalten, wobei dann mindestens eine dieser Gruppen in der vorstehend beschrieben Art modifiziert ist. Genauso können auch Mischungen von Wirkstoffen eingesetzt werden, sofern mindestens ein Wirkstoff eine Amidingruppe aufweist. Die orale Darreichungsform kann als flüssige, halbfeste oder feste Zubereitung, insbesondere als Tablette, Dragee, Pellets oder Mikrokapseln aufgemacht sein. Hierbei werden für solche Ausführungsformen, bei denen flüssige Zubereitungen eingesetzt werden, der Wirkstoff bzw. das Wirkstoffgemisch in einem geeigneten nicht toxischen Lösungsmittel, wie zum Beispiel Wasser, einwertige Alkohole, insbesondere Ethanole, mehrwertige Alkohole, insbesondere Glycerin und/oder Propandiol, Polyglykole, insbesondere Polyethylenglykole und/oder Miglyol, Glycerinformal, Dimethylisosorbit, natürliche oder synthetische Öle, aufgenommen. Für die Herstellung von halbfesten oder festen Zubereitungen gelangen die üblichen Grundmassen, wie beispielsweise Bentonit, Veegum, Guarmehl und/oder Cellulosederivate, insbesondere Methylcellulose und/oder Carboxymethylcellulose, sowie Polymere aus Vinylalkoholen und/oder Vinylpyrrolidonen, Alginate, Pektine, Polyacrylate, feste und/oder flüssige Polyethylenglykole, Paraffine, Fettalkohole, Vaseline und/oder Wachse, Fettsäuren und/oder Fettsäureester zur Anwendung.

Des Weiteren können in festen Zubereitungen die an sich bekannten Streckmittel, wie beispielsweise kolloidale Kieselsäure, Talkum, Milchzucker, Stärkepulver, Zucker, Gelatine, Metalloxide und/oder Metallsalze enthalten sein. Als weitere Zusatzstoffe bieten sich Stabilisatoren, Emulgatoren, Dispergatoren sowie Konservierungsstoffe an.

Die nach der erfindungsgemäßen Verwendung modifizierten Arzneistoffe weisen bei oraler Verabreichung eine hervorragende Resorbierbarkeit und somit Bioverfügbarkeit auf, wodurch die pharmakologische Wirkung des Amidins deutlich erhöht wird. Somit kann nunmehr eine optimale Arzneiform für die orale Anwendung von Amidinen bereitgestellt werden.

Besondere Bedeutung erlangt die erfindungsgemäße Verwendung dadurch, dass die funktionelle Gruppe Amidin wesentlicher Bestandteil von verschiedenen wichtigen Wirkstoffen für verschiedene Anwendungsgebiete ist. Die Amidin-Gruppe ist u.a. Bestandteil der folgenden Wirkstoffklassen bzw. Wirkstoffe: Proteasehemmstoffe (Thrombinhemmstoffe wie Melagatran, Hemmstoffe von Faktor Xa, Faktor VII bzw. aller Proteasen der Gerinnungskaskade; Matriptasehemmstoffe), Antikoagulantien, Thrombolytika, Antifibrinolytika, DNA- und RNA- interkalierende Verbindungen (wie Pentamidin, Diminazen, Isometamidium), N-Methyl-D-Aspartat Rezeptor-Antagonisten und Hemmstoffe viraler Enzyme (wie z. B. Neuraminidasehemmstoffe).

Wirkstoffe, die eine wirksame Amidin-Gruppe enthalten, können u.a. zur Hemmung der Blutgerinnung, zur Prophylaxe und Therapie der viszeralen und der kutanen Leishmaniose, der Trypanosomiasis (afrikanische Schlafkrankheit), der durch Pneumocystis carinii verursachten Pneumonie (PcP), zur Wachstumshemmung maligner Tumoren, Blutdrucksenkung, Neuroprotektion, sowie zur Bekämpfung von viralen Infektionen wie Influenza und von HIV-Infektionen eingesetzt werden.

Die obigen Aufzählungen sind nur beispielhaft und die Erfindung umfasst grundsätzlich alle Wirkstoffe, die mindestens eine Amidingruppe aufweisen. Die erfindungsgemäße Verwendung ist somit auf einen sehr breiten Bereich von Wirkstoffklassen und Indikationen anwendbar und kann die Bioverfügbarkeit von vielen Arzneistoffen, deren aktive Form ein Amidin enthält, deutlich erhöhen.

Als Beispiele für die erfindungsgemäß modifizierten Arzneistoffe seien das *N,N'-*Dihydroxybenzamidin und dessen erfindungsgemäßen Derivate genannt. Das *N,N'-*Dihydroxybenzamidin kann wie von Ley und Liu et al. beschrieben synthetisiert werden [Ley H. (1898) Ber. Dtsch. Chem. Ges. 31 2126-2129; Liu K.-C.; Shelton B.R.; Hews RK. (1980) J. Org. Chem. 45 3916-3918].

Zum Nachweis der Resorption aus dem Gastrointestinaltrakt und der anschließenden Reduktion zum freien Amidin wurde das *N*,*N*'-Dihydroxybenzamidin als Modellverbindung für das neue Prodrug Prinzip gewählt und drei Schweinen oral sowie intravenös verabreicht. Die Metabolisierung des *N,N'-*Dihydroxybenzamidins zu Benzamidin verläuft dabei *in vivo* folgendermaßen:

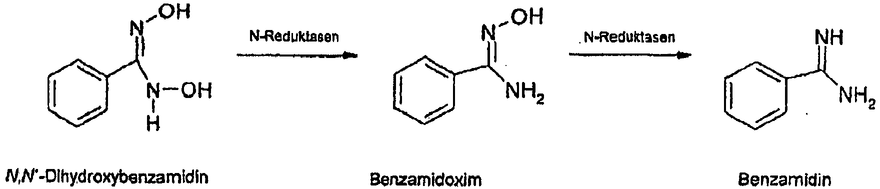

Um die genaue Dosierung der Substanzen ermitteln zu können, wurden die Tiere einmal wöchentlich gewogen. Aus den Daten wurde die tägliche Gewichtszunahme errechnet. Die oral zu verabreichenden Substanzen wurden in das angefeuchtete, gemahlene Kraftfutter gemischt. Die intravenös gegebenen Substanzen wurden in NaCl Lösung 0,9 % gelöst, um eine Hämolyse zu verhindern.

Direkt vor der Injektion in den Venenverweilkatheter wurde die Lösung filtriert, um zu verhindern, dass eventuell nicht gelöste Anteile eine Thrombenbildung induzieren. Nach der Injektion wurde noch einmal mit mindestens 10 ml NaCl Lösung 0,9 % nachgespült. Die Substanzapplikation erfolgte jeweils morgens. Am darauf folgenden Tag fand jeweils eine "washout" Periode statt, um die vollständige Ausscheidung des Arzneistoffs zu gewährleisten.

Die oral verabreichten Dosen des *N,N'*-Dihydroxybenzamidins betrugen jeweils 10 mg/kg Körpergewicht (KG). Die Konzentration der intravenös als Bolus applizierten Stoffe betrug 2 mg/kg KG. Benzamidin und das *N,N'*-Dihydroxybenzamidin wurden ebenfalls intravenös verabreicht. Die Proben wurden zu zuvor festgelegten Zeiten entnommen. Die Versuchsperiode für eine Kondition dauerte einen Tag. Die Blutproben wurden über einen Zeitraum von 24 Stunden nach Substanzgabe gewonnen. Nach oraler Gabe wurden die Proben nach 0, 30, 60, 90, 120, 150, 180, 240, 360, 480, 720 und 1440 Minuten genommen. Nach intravenöser Applikation erfolgte eine zusätzliche Probenentnahme nach 5 und 15 Minuten. Das gewonnene Vollblut wurde in Heparin Röhrchen umgefüllt und zentrifugiert (4 °C, 10 min, 1.500 g). Nach der Zentrifugation wurden ca. 4 ml Plasma als Überstand abgenommen, in Eppendorffgefäße pipettiert und bei -80 °C eingefroren. Die Plasmaproben wurden nach langsamem Auftauen 3 Minuten bei 7.000 U/min zentrifugiert, mittels Festphasenextraktion aufgearbeitet und der HPLC zugeführt.

Die Ergebnisse der Versuche sind in den Abbildungen dargestellt. Es zeigen:

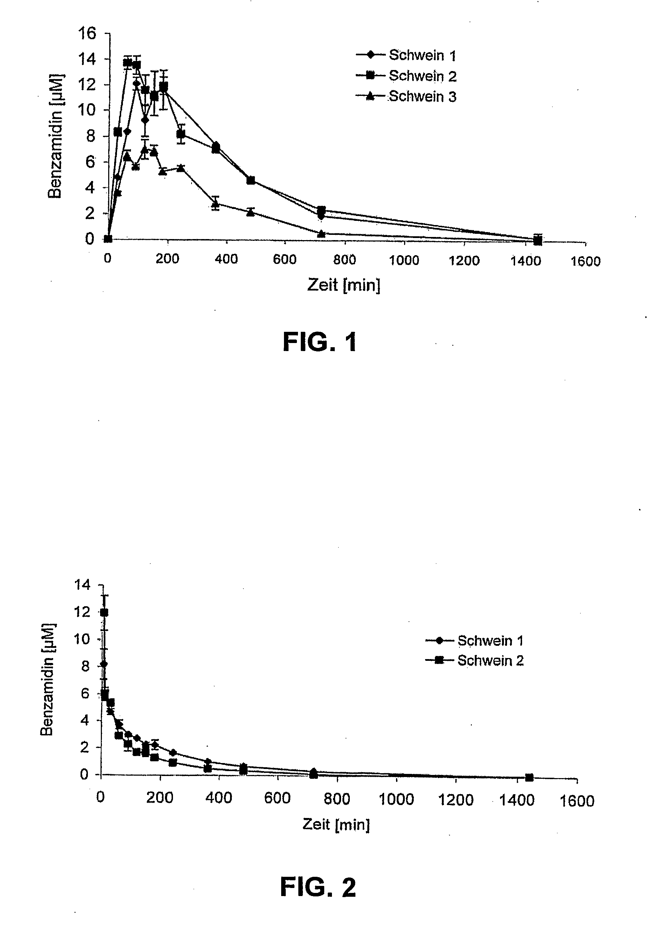

- Fig. 1: die Plasmaspiegelkurven des Benzamidins nach oraler Applikation des *N,N'-*Dihydroxybenzamidins (10 mg/kg KG) an drei Schweinen,
- Fig. 2: die Plasmaspiegelkurven des Benzamidins nach Injektion (2 mg/kg KG) an zwei Schweinen,

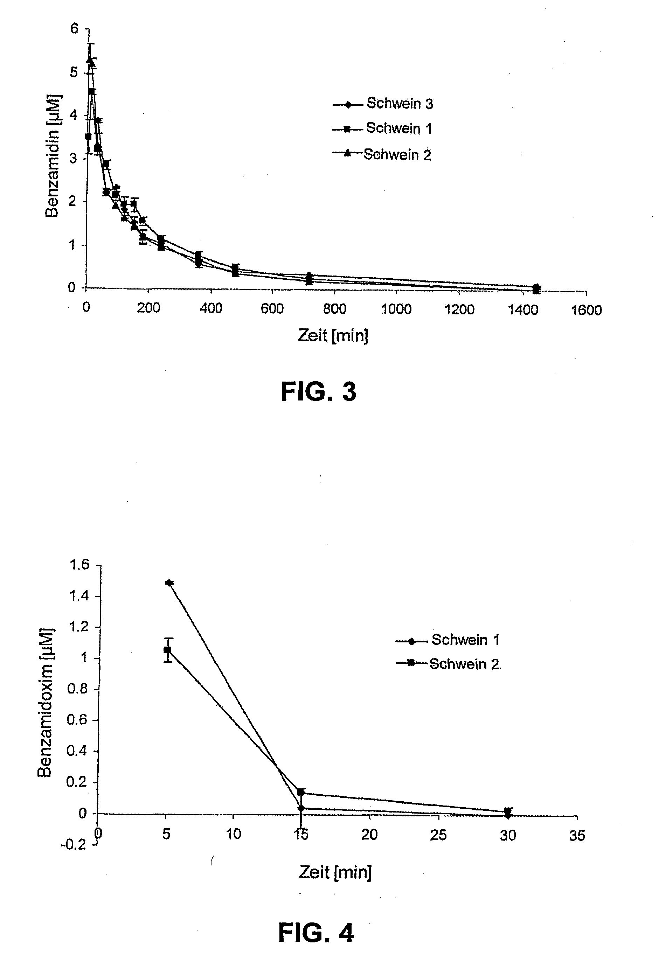

- Fig. 3: die Plasmaspiegelkurven des Benzamidins nach Injektion des *N,N'-*Dihydroxybenzamidins (2 mg/kg KG) an drei Schweinen und
- Fig. 4: die Plasmaspiegelkurven des Benzamidoxims nach Injektion des *N,N'-*Dihydroxybenzamidin (2 mg/kg KG) an zwei Schweinen.

Aus den gewonnenen Daten konnte die orale Bioverfügbarkeit des Benzamidins nach oraler Gabe des *N,N'*-Dihydroxybenzamidins bestimmt werden:

| **Tier** | **Bioverfügbarkeit** [%] | **Mittelwert** [%] | **Standardabweichung** [%] |
|---|---|---|---|
| Tier 1 | 106,71 | | |
| Tier 2 | 113,90 | 90,62 | 34,28 |
| Tier 3 | 51,25 | | |

Wie obiger Tabelle zu entnehmen ist, besitzt das Benzamidin nach oraler Gabe des *N,N'-*Dihydroxybenzamidins eine Bioverfügbarkeit von 90,62 %. Dies zeigt, dass das Prodrug nach oraler Gabe fast vollständig resorbiert und im Blut zügig zur Wirkform reduziert wird. Auch nach Injektion des *N,N'*-Dihydroxybenzamidins wird das Prodrug zügig zum Amidin reduziert, wobei nach dieser Applikationsart zusätzlich noch Benzamidoxim im Plasma detektiert werden konnte.

## Patentansprüche

1. Verwendung von N,N'-Dihydroxyamidin (I) der Formel 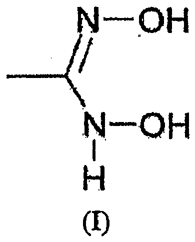 als Ersatz einer Amidinfunktion eines Arzneistoffes in Arzneimitteln zur Verbesserung der Bioverfügbarkeit des Arzneistoffes.

2. Verwendung nach Anspruch 1, **dadurch gekennzeichnet, dass** der Arzneistoff ausgewählt ist aus der Gruppe der Proteasehemmstoffe, DNA- und RNA-interkalierende Verbindungen, Hemmstoffe viraler Enzyme und N-Methyl-D-Aspartat Rezeptor-Antagonisten.

3. Verwendung nach Anspruch 2, **dadurch gekennzeichnet, dass** der Proteasehemmstoff ein Thrombinhemmstoff, ein Hemmstoff von Faktor Xa, Faktor VII bzw. aller Proteasen der Gerinnungskaskade oder ein Matriptasehemmstoff ist.

4. Verwendung nach Anspruch 2, **dadurch gekennzeichnet, dass** der Proteasehemmstoff ein Urokinasehemmstoff ist.

5. Verwendung nach Anspruch 2, **dadurch gekennzeichnet, dass** die DNA und RNA-interkalierende Verbindung Pentamidin, Diminazen oder Isometamidium ist.

6. Verwendung nach Anspruch 2, **dadurch gekennzeichnet, dass** der Hemmstoff viraler Enzyme ein Neuraminidasehemmstoff ist.

7. Verwendung nach Anspruch 2, **dadurch gekennzeichnet, dass** der Arzneistoff ein N-Methyl-D-Aspartat Rezeptor-Antagonist ist.

## Claims

1. Use of N-N'-dihydroxyamidine (I) of formula 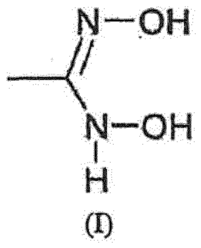 as a substitute of an amidine function of a medicinal substance in drugs for improving the bioavailability of the medicinal substance.

2. Use according to claim 1, **characterized in that** the medicinal substance is selected from the group of protease inhibitors, DNA and RNA intercalating compounds, inhibitors of viral enzymes, and N-methyl-D-aspartate receptor antagonists.

3. Use according to claim 2, **characterized in that** the protease inhibitor is a thrombin inhibitor, an inhibitor of factor Xa, factor VII or of all proteases of the coagulation cascade, or a matriptase inhibitor.

4. Use according to claim 2, **characterized in that** the protease inhibitor is an urokinase inhibitor.

5. Use according to claim 2, **characterized in that** the DNA and RNA intercalating compound is pentamidine, diminazene or isometamidium.

6. Use according to claim 2, **characterized in that** the inhibitor of viral enzymes is a neuraminidase inhibitor.

7. Use according to claim 2, **characterized in that** the medicinal substance is an N-methyl-D-aspartate receptor antagonist.

## Revendications

1. Utilisation de N,N'-dihydroxyamidine (I) de la formule 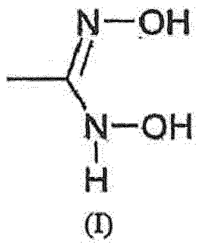 en remplacement d'une fonction d'amidine d'une substance médicamenteuse dans des médicaments visant à améliorer la biodisponibilité de la substance médicamenteuse.

2. Utilisation selon la revendication 1, **caractérisée en ce que** la substance médicamenteuse est sélectionnée dans le groupe des inhibiteurs de protéase, des liaisons d'ADN et d'ARN intercalantes, des inhibiteurs d'enzymes viraux et des antagonistes de récepteur de N-méthyle-D-aspartate.

3. Utilisation selon la revendication 2, **caractérisée en ce que** l'inhibiteur de protéase est un inhibiteur de thrombine, un inhibiteur de facteur Xa, facteur VII ou de toutes les protéases de la cascade de coagulation ou un inhibiteur de matriptase.

4. Utilisation selon la revendication 2, **caractérisée en ce que** l'inhibiteur de protéase est un inhibiteur d'urokinase.

5. Utilisation selon la revendication 2, **caractérisée en ce que** la liaison d'ADN et d'ARN intercalante est de la pentamidine, du diminazène ou de l'isométamidium.

6. Utilisation selon la revendication 2, **caractérisée en ce que** l'inhibiteur d'enzymes viraux est un inhibiteur de neuraminidase.

7. Utilisation selon la revendication 2, **caractérisée en ce que** la substance médicamenteuse est un antagoniste de récepteur de N-méthyle-D-aspartate.
